# EUROPÄISCHE PATENTSCHRIFT

(11) **EP 2 191 046 B1**
(45) Veröffentlichungstag und Bekanntmachung des Hinweises auf die Patenterteilung: **03.08.2011**
(21) Anmeldenummer: 08782856.2
(22) Anmeldetag: 15.09.2008
(51) Int. Cl.: D01F 1/02, D01F 1/10, D01F 2/00

(54) **LYOCELLFASER**
LYOCELL FIBER
FIBRE LYOCELL

(30) Priorität: 18.09.2007 AT 14572007
(43) Veröffentlichungstag der Anmeldung: 02.06.2010
(73) Patentinhaber: LENZING AKTIENGESELLSCHAFT, 4860 Lenzing (AT)
(72) Erfinder: FIRGO, Heinrich, A-4840 Vöcklabruck (AT); FUCHS, Heidrun, A-4840 Vöcklabruck (AT)
(74) Vertreter: Nemec, Harald
(86) Internationale Anmeldenummer: PCT/AT2008/000329
(87) Internationale Veröffentlichungsnummer: WO 2009/036481

(56) Entgegenhaltungen:
- WO-A-2005/017247
- DE-A1- 10 007 794

## Beschreibung

Die vorliegende Erfindung betrifft eine Lyocellfaser.

Fasern der Gattung Lyocell werden durch ein Lösungsmittelspinnverfahren hergestellt, bei welchem die Cellulose direkt ohne Ausbildung eines Derivates in einem organischen Lösungsmittel gelöst und die Lösung versponnen wird. Solche Fasern haben auch den Namen "lösungsmittelgesponnene" Fasern. "Lyocell" ist der von der BISFA (The International Bureau for the Standardization of man made fibers) vergebene Gattungsname für Cellulosefasern, welche dadurch hergestellt werden, dass Cellulose ohne Ausbildung eines Derivates in einem organischen Lösungsmittel aufgelöst wird und aus dieser Lösung Fasern mittels eines Trocken-Nass-Spinnverfahrens oder eines Melt-Blown-Verfahrens extrudiert werden. Unter einem organischen Lösungsmittel wird dabei ein Gemisch aus einer organischen Chemikalie und Wasser verstanden. Als organisches Lösungsmittel wird heute in kommerziellem Maßstab N-Methyl-Morpholin-N-Oxid eingesetzt.

Die Lösung der Cellulose wird in diesem Verfahren üblicherweise mittels eines Formwerkzeuges extrudiert und dabei ausgeformt. Die ausgeformte Lösung gelangt über einen Luftspalt in ein Fällbad, wo durch Ausfällen der Lösung der Formkörper erhalten wird. Der Formkörper wird gewaschen und ggf. nach weiteren Behandlungsschritten getrocknet. Ein Verfahren zur Herstellung von Lyocellfasern ist z.B. in der US A 4,246,221 beschrieben. Lyocellfasern zeichnen sich durch eine hohe Festigkeit, einen hohen Nassmodul und durch eine hohe Schlingenfestigkeit aus.

Es ist bekannt, Cellulosefasern und auch Lyocellfasern durch die Inkorporation verschiedenster Additive zu modifizieren.

In diesem Zusammenhang beschreibt die EP 1 259 564 B 1 Lyocellfasern, die ein Material aus Meerespflanzen oder Schalen von Meerestieren enthalten. Als Meerestiere werden auch Muscheln erwähnt. Die Schalen von Muscheln enthalten aber insbesondere alle Verunreinigungen, die sich an der Außenseite der Schale, d.h. der dem Meer zugewandten Seite, ablagern.

Die CN 1772980 A beschreibt eine Viskosefaser, welche Perlenpulver enthält.

Auch die CN 1450212 A beschreibt Fasern, welche Perlenpulver enthalten. Es handelt sich dabei aber um nicht-cellulosische Synthesefasern.

Perlenpulver (d.h. gemahlene Perlen) ist seit langem aus der chinesischen Medizin als gesundheits- und schönheitsfördernd bekannt und wird daher in kosmetischen Produkten eingesetzt. Dieses Material besteht zum Großteil aus CaCO₃ sowie aus Proteinen.

Die Herstellung von Viskosefasern und insbesondere von nicht-cellulosischen Synthesefasern unterscheidet sich deutlich von jener von Lyocellfasern, Insbesondere müssen im Viskoseverfahren zur Herstellung der Spinnlösung verschiedene umweltbelastende Chemikalien eingesetzt werden. Zudem treten in den Herstellungsverfahren dieser Fasertypen Bedingungen auf, die für die Bestandteile des Perlenpulvers schädlich sind und daher die Ausbeute verringern bzw. die Eigenschaften des Produktes verschlechtern. So kommt es bei einer Inkorporation von Perlenpulver in nicht-cellulosischen Synthesefasern zu einer schlechten Bioverfügbarkeit des Pulvers, da dieses im Inneren der Fasern eingeschlossen wird und aufgrund der Hydrophobizität solcher Fasern nur ein geringer Austausch mit der Umgebung erfolgen kann. Im Viskoseverfahren wiederum schädigt die dort durchgeführte Ausfällung der Fasern in einem sauren Fällbad, welches H₂SO₄ enthält, das im Perlenpulver vorhandene CaCO₃. Es kommt jedenfalls zu einer partiellen Auflösung des enthaltenen CaCO₃, und es ist auch eine teilweise Ausfällung als CaSO₄ zu erwarten.

Auch die Eigenschaften der verschiedenen Fasertypen weichen deutlich voneinander ab.

Die vorliegende Erfindung stellt sich zur Aufgabe, eine Faser zur Verfügung zu stellen, in der reines Perlenmaterial mit hoher Bioverfügbarkeit vorliegt und welche mit einem für das Perlenmaterial möglichst schonenden Verfahren hergestellt werden kann.

Diese Aufgabe wird mit einer Lyocellfaser, enthaltend ein Material ausgewählt aus der Gruppe bestehend aus jeweils ausschließlich aus dem Hypostracum von Muscheln gewonnenem Perlenpulver, gemahlenem Perlmutt und Mischungen davon, gelöst.

Die erfindungsgemäße Faser enthält somit ein hochreines Material aus Perlen bzw. aus Perlmutt. Beim Perlmutt und bei den Perlen handelt es sich ausschließlich um die innerste dem Mollusk zugewandte Schicht, dem Hypostracum. Es besteht zu 95% aus pseudohesagonalen Calciumcarbonatplättchen von 500 nm Dicke in der kristallinen Form des Aragonits. Die Plänchen sind in eine organische Matrix aus Proteinen und Chitin eingebettet. Perlen bestehen im Wesentlichen aus Perlmutt mit 80-95% aus CaCO3, das in der kristallinen Form des Aragonits, mit möglicherweise geringem Anteil Calcit vorliegt (http://de.wikipedia.org/wiki/Perle; http-//de.wikipedia.org/wiki/Perlmutt). Abgeschlossen im Inneren der Muschel(schale) gebildet, entsteht die Perle geschützt vor schädlichen Umwelteinflüssen und daher (im Gegensatz zu ganzen Muschelschalen) ohne Verunreinigung.

Das Material aus Perlen bzw. Perlmutt kann bevorzugt in einem Anteil von 0,07 Gew.% bis 5 Gew.%, bezogen auf Faser, vorliegen.

Insbesondere bevorzugt handelt es sich bei dem Material um ein hochfeines Pulver, welches eine durchschnittliche Partikelgröße von 0,04 bis 1,5 µm, besonders bevorzugt 0,4 bis 1,0 µm, aufweist. Solche Pulver sind unter der Bezeichnung "Nano-Pearl-Powder" kommerziell erhältlich bzw. können durch entsprechende Mahlung von Perlen und/oder Perlmutt hergestellt werden.

Das Verfahren zur Herstellung von Lyocellfasern beruht bekanntermaßen gegenüber dem Verfahren zur Herstellung von Viskosefaser auf einer Auflösung der Cellulose ohne vorhergehende Derivatisierung und kann sehr umweltschonend durchgeführt werden.

Das Verfahren zur Herstellung der erfindungsgemäßen Lyocellfaser enthält daher in an sich bekannter Weise die Schritte
- Herstellung einer Spinnlösung von Cellulose in einem wässerigen tertiären Aminoxid, bevorzugt N-methylmorpholin-N-oxid (NMMO)
- Verspinnen der Spinnlösung zu Fasern,
   und ist dadurch gekennzeichnet, dass ein Material ausgewählt aus der Gruppe bestehend aus jeweils ausschließlich aus dem Hypostracum von Muscheln gewonnenem Perlenpulver, gemahlenem Perlmutt und Mischungen davon in die Spinnlösung und/oder einem Vorläufer davon eingemischt wird.
   Unter dem "Vorläufer" der Spinnlösung sind Ausgangs- und Zwischenmaterialien der Herstellung der Spinnlösung zu verstehen, insbesondere
- das verwendete Ausgangs-Cellulosematerial, z.B. Zellstoff
- das verwendete Lösungsmittel (wässeriges tertiäres Aminoxid, in der Folge stellvertretend für alle verwendbaren Aminoxide mit "NMMO" abgekürzt)
- eine Mischung des Ausgangs-Cellulosematerials mit dem Lösungsmittel NMMO, insbesondere eine Suspension des Cellulosematerials in einem wässerigen NMMO, aus welcher die Lösung hergestellt werden kann.

Das Material aus Perlen und/oder Perlmutt kann bevorzugt in einem Anteil von 0,1 Gew.% bis 5 Gew.% (bezogen auf Cellulose) eingemischt werden. Typische Ausbeuteverluste liegen im Bereich von 5 bis 30%, insbesondere bei lediglich ca. 10%.

Das Material wird bevorzugt in Form eines Pulvers eingesetzt. Bevorzugt liegt die durchschnittliche Partikelgröße des Pulvers von 0,04 bis 1,5 µm, besonders bevorzugt 0,4 bis 1,0 µm.

Vor der Zugabe zur Spinnlösung bzw. zum Vorläufer davon kann das Pulver bevorzugt in Form einer wässerigen Suspension gebracht werden. Diese Suspension wird dann zugemischt.

Die vorliegende Erfindung betrifft auch die Verwendung der erfindungsgemäßen Lyocellfaser als Produkt mit kosmetischer Wirkung in einer Textilware und/oder in einem nicht-gewebten Kosmetikprodukt.

Perlenpulver wird eine die Haut weichmachende, Hautzellen erneuernde, die Melaninsythese und damit die Ausbildung von Altersflecken unterdrückende und z.T. auch eine antioxidative Wirkung nachgesagt. Das Wirkungsspektrum ist auf die topische Applikation bioverfügbarer essentieller Aminosäuren und Spurenelemente zurückzuführen.

Für die Anwendung als Kosmetikum ist nachgewiesen, dass Partikelgrößen im Nano-Bereich (40-80nm) gegenüber Micrometer großen Partikeln die Absorptionsgeschwindigkeit von Ca und Aminosäuren durch die Haut erhöhen (http://www.karipearl.com/medicine.html).

Die Reinheit und die spezielle Zusammensetzung aus essentiellen Aminosäuren, Mineralstoffen, Spurenelementen und dem hohen Ca-Anteil machen die Perle bzw. das Perlmutt für die Haut besonders wertvoll.

Das in die Lyocellfaser inkorporierte Material aus Perlen und/oder Perlmutt kann über einen Slow-Release-Effekt Mineralstoffe, essentielle Aminosäuren und Calcium ständig in kleinen Dosen an die Haut abgeben. Die Haut wird somit ständig mit diesen Micronutrients versorgt.

### Beispiele:

Prinzipielle Methode:
Die Herstellung der erfindungsgemäßen Faser kann durch Einmischen von 0,5-5% eines Pulvers aus gemahlenen Perlen (im folgenden: "Nano Pearl Powder" genannt) in die Lyocell-Spinnmasse und Ausspinnen unter Einstellung der üblichen Spinnparameter erfolgen. Es hat sich gezeigt, dass das Einspinnen keinen signifikanten Einfluss auf die Stabilität der Spinnmasse hat.

### Beispiel 1: Einspinnen an einer Davenport-Apparatur

Spinnmasseherstellung:
3 Gew.% (bezogen auf die Masse der Cellulose) Nano Pearl Powder wurden in eine Spinnmasse wie folgt eingemengt: 0,78 g Nano Pearl Powder (Hersteller: Fenix, Partikelgröße laut Herstellerangaben: 40 nm bis 80 nm) wurden in 5 ml VE-Wasser im Ultraschallbad aufgeschlämmt.

Bei der Lösungsherstellung wurden zuerst ca. 50%iges wässriges NMMO in an sich bekannter Weise mit einem Stabilisator (0,1 %) versetzt, danach die Aufschlämmung von Nano-Pearl Powder zugegeben und im Anschluss der Zellstoff. Die Masse wurde 1 h bei Raumtemperatur und 250 mbar suspendiert, auf 70°C erwärmt und danach die Lösung in an sich bekannter Weise durch Abdampfen von Wasser hergestellt und nachgewärmt. Die auf diese Weise hergestellte Lösung ist optisch klar und frei von Partikeln > 3 µm.

Die hergestellte Lösung enthielt 77% NMMO, 13% Cellulose, 10% Wasser, 0,1% Stabilisator (alles bezogen auf die Masse der Lösung) und 3 Gew.% Nano-Pearl-Powder (bezogen auf die Masse der Cellulose).

Die auf diese Weise hergestellte Spinnmasse wurde an einer Davenport-Spinnapparatur durch eine Spinndüse auf an sich bekannte Weise bei 115°C über einen Luftspalt ausgesponnen. Die verwendete Spinndüse hatte einen Lochdurchmesser von 100 µm.

Die hergestellten Fasern wurden auf an sich bekannte Weise gewaschen und geschnitten.

Die hergestellte Faser wies einen Titer von 1,35 dtex auf und hatte zufriedenstellende Festigkeitseigenschaften.

Der Rohstoff (Nano Pearl Powder) und die daraus erzeugte Faser hatten folgende Analysendaten:

| **Probe** | **Al** [mg/kg] | **Ca** [mg/kg] | **Fe** [mg/kg] | **Mg** [mg/kg] | **Mn** [mg/kg] | **Na** [mg/kg] | **Ni** [mg/kg] | **P** [mg/kg] | **S** [mg/kg] | **Si** [mg/kg] | **Zn** [mg/kg] |
|---|---|---|---|---|---|---|---|---|---|---|---|
| Nano-Powder | 290 | 400000 | 450 | 43 | 740 | 3100 | 48 | 140 | 240 | 4000 | 3,9 |
| Faser | 4,4 | 7100 | 17 | 9,2 | 12 | 47 | 1,3 | 3,4 | 44 | 82 | 4,4 |

### Beispiel 2: Einspinnen am Spinntechnikum

Es wurde eine Spinnlösung mit folgender Zusammensetzung hergestellt:
13% Zellstoff
10,5% H₂O
76,5% NMMO
3% Nano Pearl Powder (bezogen auf Masse der Cellulose)

Die Spinnmasse enthielt zudem einen Stabilisator, wie an sich bekannt.

Das Nano Pearl Powder wurde zuerst in 78%igem NMMO mit einen Ultra Turrax T 50 dispergiert. Dann wurde der Zellstoff zugegeben, eine Suspension bereitet und daraus in an sich bekannter Weise eine Lösung hergestellt.

Die fertige Lösung wurde bei einer Spinntemperatur von 120°C versponnen.

Die hergestellte Faser wies einen Titer von 1,35 dtex auf und hatte zufriedenstellende Festigkeitseigenschaften.

### Bilanz der Ausbeute an Calcium:

Im verwendeten Nano-Pearl-Powder befinden sich 40% Calcium. Es wurde ein Verlust von Calcium in den Waschwässern von 12,7% festgestellt.

## Patentansprüche

1. Lyocellfaser, enthaltend ein Material ausgewählt aus der Gruppe bestehend aus jeweils ausschließlich aus dem Hypostracum von Muscheln gewonnenem Perlenpulver, gemahlenem Perlmutt und Mischungen davon.

2. Lyocellfaser gemäß Anspruch 1, **dadurch gekennzeichnet, dass** das Material in einem Anteil von 0,07 Gew.% bis 5 Gew.% bezogen auf Faser vorliegt.

3. Lyocellfaser gemäß Anspruch 1 oder 2, **dadurch gekennzeichnet, dass** das Material eine durchschnittliche Partikelgröße von 0,04 bis 1,5 µm, bevorzugt 0,4 bis 1,0 µm, aufweist.

4. Verfahren zur Herstellung einer Lyocellfaser gemäß einem der vorherigen Ansprüche, enthaltend die Schritte
- Herstellung einer Spinnösung von Cellulose in einem wässerigen tertiären Aminoxid, bevorzugt N-methylmorpholin-N-oxid (NMMO)
- Verspinnen der Spinnlösung zu Fasern,
**dadurch gekennzeichnet, dass** ein Material ausgewählt aus der Gruppe bestehend aus jeweils ausschließlich aus dem Hypostracum von Muscheln gewonnenem Perlenpulver, gemahlenem Perlmutt und Mischungen davon in die Spinnlösung und/oder einem Vorläufer davon eingemischt wird.

5. Verfahren gemäß Anspruch 4, **dadurch gekennzeichnet, dass** das Material in einem Anteil von 0,1 Gew.% bis 5 Gew.% (bezogen auf Cellulose) eingemischt wird.

6. Verfahren gemäß Anspruch 4 oder 5, **dadurch gekennzeichnet, dass** das Material in Form eines Pulvers mit einer durchschnittlichen Partikelgröße von 0,04 bis 1,5 µm, bevorzugt 0,4 bis 1,0 µm, eingesetzt wird.

7. Verfahren gemäß Anspruch 6, **dadurch gekennzeichnet, dass** das Pulver vor der Zugabe zur Spinnlösung bzw. zum Vorläufer davon in Form einer wässerigen Suspension gebracht wird.

8. Verwendung einer Lyocellfaser gemäß einem der Ansprüche 1 bis 3 als Produkt mit kosmetischer Wirkung in einer Textilware und/oder in einem nicht-gewebten Kosmetikprodukt.

## Claims

1. Lyocell fibre, containing a material selected from the group consisting of pearl powder, ground nacre and mixtures thereof, said material having being obtained exclusively from the hypostracum of mussles.

2. Lyocell fibre according to claim 1, **characterized in that** the material is present in an amount of from 0.07 wt.% to 5 wt.%, based on fibre.

3. Lyocell fibre according to claim 1 or 2, **characterized in that** the material has an average particle size of from 0.04 to 1.5 µm, preferably 0.4 to 1.0 µm.

4. Process for the manufacture of a Lyocell fibre according to any of the preceding claims, comprising the steps of
- manufacturing a spinning solution of cellulose in an aqueous tertiary amine oxide, preferably N-methylmorpholine-N-oxide (NMMO)
- spinning the spinning solution to fibres,
**characterized in that** a material selected from the group consisting of pearl powder, ground nacre and mixtures thereof, said material having being obtained exclusively from the hypostracum of mussles, is admixed to the spinning solution and/or to a precursor thereof.

5. Process according to claim 4, **characterized in that** the material is admixed in an amount of from 0.1 wt.% to 5 wt.%, based on cellulose.

6. Process according to claim 4 or 5, **characterized in that** the material is employed in the form of a powder with an average particle size of from 0.04 to 1.5 µm, preferably 0.4 to 1.0 µm.

7. Process according to claim 6, **characterized in that** the powder is converted into the form of an aqueous suspension prior to being admixed to the spinning solution or the precursor thereof, respectively.

8. Use of a Lyocell fibre according to any of claims 1 to 3 as a product with cosmetic effect in a textile article and/or in a non-woven cosmetic product.

## Revendications

1. Fibres Lyocell contenant un matériau sélectionné dans l'ensemble constitué de poudre de perle obtenue exclusivement à partir de l'hypostracum de coquillages, de nacre broyée et de leurs mélanges.

2. Fibres Lyocell selon la revendication 1, **caractérisées en ce que** le matériau est présent en une proportion de 0,07 % en poids à 5 % en poids par rapport aux fibres.

3. Fibres Lyocell selon les revendications 1 ou 2, **caractérisées en ce que** le matériau présente des particules d'une taille moyenne de 0,04 à 1,5 µm et de préférence de 0,4 à 1,0 µm.

4. Procédé de fabrication de fibres Lyocell selon l'une des revendications précédentes, et contenant les étapes qui consistent à :
préparer une solution de filage de cellulose dans une solution aqueuse d'un oxyde d'amine tertiaire, de préférence le N-méthylmorpholine N-oxyde (NMMO) et
filer la solution de filage en fibres,
**caractérisé en ce que**
un matériau choisi dans le groupe constitué de poudre de perle obtenue exclusivement à partir de l'hypostracum de coquillages, de nacre broyée et de leurs mélanges est incorporé dans la solution de filage et/ou un précurseur de cette dernière.

5. Procédé selon la revendication 4, **caractérisé en ce que** le matériau est incorporé en une proportion de 0,1 % en poids à 5 % en poids (par rapport à la cellulose).

6. Procédé selon les revendications 4 ou 5, **caractérisé en ce que** le matériau est utilisé sous la forme d'une poudre de particules d'une taille moyenne de 0,04 à 1,5 µm et de préférence de 0,4 à 1,0 µm.

7. Procédé selon la revendication 6, **caractérisé en ce que** la poudre est mise en suspension aqueuse avant d'être ajoutée à la solution de filage ou à un précurseur de cette dernière.

8. Utilisation d'une fibre Lyocell selon l'une des revendications 1 à 3 comme produit à action cosmétique dans un produit textile et/ou un produit cosmétique non tissé.
